# EUROPÄISCHE PATENTSCHRIFT

(11) **EP 1 868 491 B1**
(45) Veröffentlichungstag und Bekanntmachung des Hinweises auf die Patenterteilung: **18.05.2011**
(21) Anmeldenummer: 06725233.8
(22) Anmeldetag: 22.03.2006
(51) Int. Cl.: A61B 5/00, A61B 5/15, A61M 5/32

(54) **ANALYTISCHES HILFSMITTEL MIT LANZETTE UND TESTELEMENT**
ANALYZING MEANS WITH LANCET AND TEST ELEMENT
MOYEN D'ANALYSE AVEC LANCETTE ET ELEMENT D'ESSAI

(30) Priorität: 24.03.2005 EP 05006631
(43) Veröffentlichungstag der Anmeldung: 26.12.2007
(73) Patentinhaber: Roche Diagnostics GmbH, 68305 Mannheim (DE); F. Hoffmann-La Roche AG, 4070 Basel (CH)
(72) Erfinder: LIST, Hans, 64754 Hesseneck-Kailbach (DE)
(74) Vertreter: Stößel, Matthias
(86) Internationale Anmeldenummer: PCT/EP2006/060950
(87) Internationale Veröffentlichungsnummer: WO 2006/100265

(56) Entgegenhaltungen:
- EP-A- 0 088 257
- EP-A- 1 508 304
- WO-A-2004/066822
- DE-A1- 2 909 349

## Beschreibung

Die Erfindung betrifft ein analytisches Hilfsmittel, das eine Lanzette und ein Testelement enthält, ein Analysegerät zur Analyse einer Probe mittels eines solchen analytischen Hilfsmittels.

Die Untersuchung von Blutproben oder von interstitieller Flüssigkeit ermöglichen in der klinischen Diagnostik das frühzeitige und zuverlässige Erkennen von pathologischen Zuständen sowie die gezielte und fundierte Kontrolle von Körperzuständen. Die medizinische Diagnostik setzt stets die Gewinnung einer Probe aus Blut oder interstitieller Flüssigkeit des zu untersuchenden Individuums voraus.

Zur Gewinnung der Probe kann die Haut z.B. an der Fingerbeere oder dem Ohrläppchen der zu untersuchenden Person mit Hilfe einer sterilen, scharfen Lanzette perforiert werden, um so einige wenige Mikroliter Blut oder weniger für die Analyse zu gewinnen. Insbesondere eignet sich diese Methode für die Analyse einer Probe, die unmittelbar nach der Probengewinnung durchgeführt wird.

Vor allem im Bereich des so genannten "Home-Monitoring", also dort, wo medizinische Laien selbst einfache Analysen des Bluts oder der interstitiellen Flüssigkeit durchführen, und dort insbesondere für die regelmäßige, mehrmals täglich durchzuführende Blutgewinnung durch Diabetiker für die Kontrolle der Blutglukosekonzentration, werden Lanzetten und dazu passende Geräte (so genannte Stechhilfen), angeboten, die eine möglichst schmerzarme und reproduzierbare Probengewinnung ermöglichen. Solche Lanzetten und Geräte (Stechhilfen) sind z.B. Gegenstand von WO-A 98/48695, EP-A 0 656 970, US 4,442,836 oder US 5,554,166.

Die eigenhändige Blutzuckerbestimmung ist heute eine weltweit verbreitete Methode in der Diabetes-Kontrolle. Blutzuckergeräte im Stand der Technik bestehen aus einem Analysegerät, in das ein Testelement (Teststreifen) eingeführt wird. Das Testelement wird mit einem Tropfen einer Probe in Kontakt gebracht, der zuvor mittels einer Stechhilfe z.B. aus der Fingerbeere gewonnen wurde.

Die zahlreichen Systemkomponenten (Lanzette, Stechhilfe, Testelement und Analysegerät) benötigen viel Platz und bedingen eine retativ komplexe Handhabung. Inzwischen gibt es auch Systeme mit einem höhere Grad der Integration und damit einer einfacheren Handhabung, bei denen z.B. die Testelementes im Analysengerät magaziniert und für die Messung zur Verfügung gestellt werden. Ein nächster Schritt der Miniaturisierung ist beispielsweise durch die Integration von mehreren Funktionen bzw. Funktionselementen in einem einzigen analytischen Hilfsmittel (Disposable) zu erreichen. Durch geeignete Kombination von Stechvorgang und sensorischer Analytkonzentrations-Erfassung auf einem Testelement lässt sich beispielsweise der Bedienablauf deutlich vereinfachen.

DE-A1 103 15 544 bezieht sich auf ein Verfahren zur Herstellung von kombinierten Stech- und Messvorrichungen zum Nachweis eines Analysen in einer Flüssigkeit, die einen Träger und ein Nachweiselement enthalten. Das Verfahren umfasst die Verfahrensschritte des Erzeugens von Stechspitzen definierende Ausnehmungen an einer Stirnseite des bandförmigen Trägermaterials, des Aufbringens eines Nachweiselementes auf das bandförnige Trägermaterial und des einzelnen oder paketweise erfolgenden Abtrennen von Einzel-Stech-Mess-Disposable-Grundkörpern vom bandförmigen Trägermaterial an Trennlinien. Die so hergestellten kombinierten Stech- und Messvorrichtungen enthalten demnach Einzel-Stech-Mess-Disposable-Grundkörper, die eine Stechspitze aufweisen. Die Stechspitze ist mit einer Weichplastik-Abdeckung versehen. Der Grundkörper umfasst ferner ein Nachweiselement, welches nach einer Sterilisierung und/oder Versiegelung des Einzel-Stech-Mess-Disposable-Grundkörpers auf diesem aufgebracht ist.

DE-A1 101 42 232 hat ein analytisches Hilfsmittel mit Lanzette und Testelement zum Gegenstand. Die Lanzette umfasst eine Lanzettennadel mit einer Spitze und einen Lanzettenkörper, der die Lanzettennadel zumindest im Bereich der Spitze vollständig umgibt, wobei die Lanzettenttadel relativ zum Lanzettenkörper verschiebbar ist. Der Lanzettenkörper besteht zumindest im Bereich der Spitze der Lanzettennadel aus einem elastischen Material, in das die Spitze der Lanzettennadel eingebettet ist. Das analytische Testelement ist fest mit dem Lanzettenkörper verbunden.

EP-A1 1 508 304 offenbart ein verpacktes medizinisches Gerät. Dieses umfasst eine entfaltbare Lanzette. Weiterhin umfasst das Gerät eine obere flexible Schicht, einen Teststreifen sowie eine untere flexible Schicht.

Ein häufig im Stand der Technik zu findender Ansatz für solche analytischen Hilfsmittel ist, die Lanzette mit einer Kapillarstruktur zu versehen oder sie zu einem Teil einer Kapillarstruktur zu machen, so dass die Lanzettenspitze nach dem Einstich und dem Blutsaustritt an das Blut herangeführt wird, um dieses mit der Kapillare zu einer auf einem Testelement vorhandenen Testchemie zu befördern. Dieser Ansatz hat jedoch einige Nachteile.

Die Lanzettenspitze ist bis unmittelbar vor Gebrauch steril zu halten. Für den Gebrauch muss der Sterilschutz entfernt werden. Danach darf er nicht die Kapillarfunktion behindern, sondern muss vollständig aus dem Blutweg entfernt sein. Der Sterilschutz muss gegebenenfalls abgenommen werden, was zu einem Einzelteil führt, dessen maschinelle Behandlung kompliziert ist.

Die Kapillarstruktur, die zumindest zum Teil aus der Lanzettenspitze besteht, muss hydrophil sein, damit der Bluttransport funktionieren kann. Der Sterilschutz muss aber bis zur Benutzung der Lanzette dicht abschließen, um ein Hineinwandern von Keimen zu unterbinden. Es ist also ein inniger Kontakt zwischen Sterilschutz und Lanzettenspitze oder Lanzettenschaft notwendig. Dieser innige Kontakt kann jedoch die Oberflächeneigenschaften, insbesondere die Hydrophilie, der Lanzette nachteilig verändern.

Nach dem Blutsaustritt muss die Öffnung der Kapillare genau die kleine Blutprobe treffen, damit diese aufgesaugt werden kann. Dabei besteht die Gefahr, dass die scharfe Spitze der Lanzette nochmals in das Gewebe einsticht. Es muss also eine genaue Kenntnis der Position der Hautoberfläche vorliegen.

Die Herstellung von analytischen Hilfsmitteln birgt eine Vielzahl zu lösender Probleme. Die Lanzette ist zu sterilisieren und die Lanzettensterilität für den Zeitraum der Aufbrauchfrist des analytischen Hilfsmittels sicherzustellen. Die in dem Testfeld des Testelements vorhandene Testchemie kann durch die bekannten Verfahren zur Sterilisierung nachteilig in ihrer Funktions- und Wirkungsweise beeinflusst werden. Es können empfindliche chemische oder biologische Substanzen geschädigt werden. Die Testchemie sollte daher möglichst nicht dem Sterilisierungsverfahren für die Lanzette ausgesetzt werden.

Die Aufgabe der vorliegenden Erfindung war es daher, die Nachteile des Standes der Technik zu vermeiden und die genannten Probleme zu lösen.

Diese Aufgabe wird erfindungsgemäß gelöst durch ein analytisches Hilfsmittel, enthaltend einen Grundkörper, eine Lanzette und ein Testelement, dadurch gekennzeichnet,
i) dass die Lanzette ein proximales und ein distales Ende aufweist, wobei das proximale Ende der Lanzette als Lanzettenspitze ausgebildet ist und das distale Ende der Lanzette fest mit dem Grundkörper verbunden ist und wobei zumindest die Lanzettenspitze in einer Ausnehmung in dem Grundkörper eingebettet und die Ausnehmung mit einer Abdeckung verschlossen ist, so lange sich das analytische Hilfsmittel in einem unbenutzten Zustand befindet und
ii) dass der Grundkörper eine Sollbruchstelle aufweist, an der der Grundkörper bei Aufbringen einer Spannung in zwei Teilkörper zerbricht, wobei die Sollbruchstelle so angeordnet ist, dass beim Brechen des Grundkörpers an der Sollbruchstelle die Lanzettenspitze zur Benutzung freigegeben wird und
iii) dass das Testelement fest mit dem Grundkörper verbunden ist.

Bei diesem analytischen Hilfsmittel (oder synonym dazu "Disposable") werden die drei Funktionen Einstechen, Probenaufnahme und Bereitstellen einer Testchemie zur Analyse der Probe zusammengefasst.

Der Grundkörper des analytischen Hilfsmittels dient als Träger für die Lanzette und das Testelement und ist vor Gebrauch einstückig und vorzugsweise streifenförmig ausgebildet.

Die Lanzettenspitze dient zum Perforieren der Haut eines Patienten zur Gewinnung einer Blutsprobe oder einer Probe aus interstitieller Flüssigkeit. Durch das Einbetten der Lanzette, insbesondere der Lanzettenspitze, in der Ausnehmung in den Grundkörper und durch das Verschließen der Ausnehmung mit der Abdeckung kann zumindest die Lanzettenspitze im unbenutzten Zustand bis unmittelbar vor der Benutzung steril (keimfrei) gehalten werden. Die zwei Komponenten des analytischen Hilfsmittels des Grundkörpers mit Lanzette einerseits und des Testelements andererseits können getrennt voneinander vorkonfektioniert werden. Das Sterilisieren kann daher vor dem Anbringen des Testelements an dem Grundkörper erfolgen (beispielsweise durch β- oder γ-Bestrahlung), so dass die Testchemie nicht der Sterilisierung ausgesetzt wird.

Der Grundkörper weist eine Sollbruchstelle auf. Diese Sollbruchstelle ist so konzipiert, dass der zumindest im Bereich der Sollbruchstelle spröde ausgebildete Grundkörper beim Aufbringen einer Biegespannung an dieser Sollbruchstelle in zwei Teilkörper zerbricht. Sprödigkeit bedeutet in diesem Zusammenhang insbesondere, dass das Material eine kleine Bruchdehnung aufweist. Vorzugsweise weist das Material außerdem eine geringe Kerbschlagfähigkeit auf. Ein bevorzugtes Material ist z.B. Polymethylmethacrylat.

Die Position der Sollbruchstelle ist so gewählt, dass die Lanzettenspitze beim Zerbrechen des Grundkörpers freigegeben wird, insbesondere dass die Lanzettenspitze über die Bruchkante des ersten Teilkörpers hinausragt.

Das Testelement ist mit dem Grundkörper vorzugsweise durch ein bekanntes Verfahren fest verbunden, z.B. durch Verkleben, Verschweißen, Verklipsen oder Verschrauben. Dabei ist das Testelement bevorzugt an zwei Enden fest mit dem Grundkörper verbunden, besonders bevorzugt das erste Ende des Testelements mit dem ersten Teilkörper und das zweite Ende des Testelements mit dem zweiten Teilkörper.

Gemäß einer bevorzugten Ausführungsform der vorliegenden Erfindung ist die Ausnehmung in dem Grundkörper ein in Längsrichtung verlaufender länglicher Schlitz.

Die Lanzette umfasst gemäß einer bevorzugten Ausführungsform der vorliegenden Erfindung eine messerartige Klinge (Flachlanzette), die sich zur Lanzettenspitze hin verjüngt. Diese messerartige Klinge wird in der vorzugsweise als Schlitz ausgebildeten Ausnehmung in dem Grundkörper versenkt. Beim Zerbrechen des Grundkörpers an der Sollbruchstelle durchstößt oder durchschneidet die messerartige Klinge der Lanzette die Abdeckung der Ausnehmung mit ihrer schmalen (gegebenenfalls scharf geschliffenen) Seite, wobei sie in Schneiderichtung aufgrund der Breite der Klinge eine erhöhte Steifigkeit aufweist.

Eine bevorzugte Ausführungsform der vorliegenden Erfindung ist so gestaltet, dass die Lanzette einen das proximale Ende umfassenden ersten Teilabschnitt und einen mit dem distalen Ende verbundenen zweiten Teilabschnitt enthält, wobei der erste Teilabschnitt abgewinkelt zu dem zweiten Teilabschnitt ausgebildet ist. Vorzugsweise ist der zweite Teilabschnitt der Lanzette mit dem Grundkörper fest verbunden. Besonders bevorzugt ist der erste Teilabschnitt in einem Winkel von 90° zu dem zweiten Teilabschnitt angeordnet. Der erste Teilabschnitt (z.B. in Form einer messerartigen Klinge) kann dann in einer schlitzförmigen Ausnehmung zu liegen kommen, während der senkrecht dazu angeordnete zweite Teilabschnitt auf der senkrecht zu dem Schlitz verlaufenden Oberfläche des Grundkörpers aufliegen und mit dieser fest verbunden sein kann.

Die Abdeckung umfasst vorzugsweise mindestens eine Folie, die mindestens eine Oberfläche des Grundkörpers ganz oder teilweise abdeckt. Die Folien sind möglichst dünn. Sie sind gegebenenfalls auf der Ober- und der Unterseite des Grundkörpers angebracht. Das Material der Folien sollte möglichst mit dem Material des Grundkörpers kompatibel sein, d.h. z.B. mit dem Grundkörper verschweißbar sein. Es sollte zum Gebrauch des analytischen Hilfsmittels bereitwillig in einem dafür vorgesehenen Bereich reißen. Ein Bruch im Grundkörper kann dabei als Kerbe dienen, die ein Reißen der Folie auslöst. Bevorzugte Materialien der Abdeckung sind beispielsweise Polystyrol oder Zellophan. Die Folien weisen bevorzugt eine Dicke zwischen 2 und 20 um, besonders bevorzugt zwischen 5 und 15 um auf.

Die Abdeckung kann z.B. durch Verkleben oder Verschweißen mit dem Grundkörper verbunden sein. Insbesondere im Bereich der Sollbruchstelle ist die Abdeckung mit dem Grundkörper des erfindungsgemäßen analytischen Hilfsmittels fest verbunden, damit sie beim Zerbrechen des Grundkörpers in zwei Teilkörper ebenfalls aufgetrennt wird und sich nicht vorzeitig von dem Grundkörper ablöst.

Das Material des Grundkörpers (insbesondere im Bereich der Sollbruchstelle) ist vorzugsweise ein Kunststoff.

Gemäße einer Variante der vorliegenden Erfindung bestehen der Grundkörper und/oder die Abdeckung aus einem Material, das durch Bestrahlung versprödet. Beispielsweise können der Grundkörper ein Kunststoffträger und die Abdeckung einer Anzahl von Folien sein, die durch die Einwirkung von γ-Strahlung stark verspröden. Dies erlaubt eine Fertigung des Grundkörpers und/oder der Abdeckung von der Rolle, auf der die Rohbauteile von Grundkörper und Abdeckung aufgrund ihrer ursprünglichen Flexibilität aufwickelbar sind. Nach der Fertigung erfolgt die Versprödung des Grundkörpers und/oder der Abdeckung durch die Bestrahlung, so dass beim Gebrauch der so gefertigten analytischen Hilfsmittel trotzdem ein einfaches Auseinanderbrechen des Grundkörpers und/oder der Abdeckung gewährleistet ist. Ein mögliches Material für diese Ausführungsvariante ist Polystyrol.

Gemäß einer weiteren bevorzugten Variante der vorliegenden Erfindung wird der Grundkörper des erfindungsgemäßen analytischen Hilfsmittels als (insbesondere symmetrisches) Spritzgussteil hergestellt.

Die Sollbruchstelle des Grundkörpers liegt vorzugsweise als seitliche Einkerbungen und/oder als mindestens eine Querrille im Grundkörper vor.

Gemäß einer bevorzugten Ausführungsform der vorliegenden Erfindung besteht das Testelement zumindest teilweise aus einem flexiblen Material. Flexibel bedeutet in diesem Zusammenhang, dass das Material des Testelements ausreichend verformbar ist, um vor der Fertigung des erfindungsgemäßen analytischen Hilfsmittels auf einer Rolle aufwickelbar zu sein, um vor Gebrauch in Form von mindestens einer Schlaufe an dem Grundkörper befestigbar zu sein und um beim Gebrauch über ein Bauteil des Analysegeräts, in dem das analytische Hilfsmittel verwendet wird, spannbar zu sein, so dass sich das Testelement an die Oberfläche des Bauteils anschmiegt. Das Testelement kann beispielsweise ein Abschnitt eines flexiblen Testbandes sein, der ein Testfeld enthält. Vorzugsweise ist das Testelement an zwei Enden fest mit dem Grundkörper verbunden.

Eine bevorzugte Ausführungsform der vorliegenden Erfindung ist so gestaltet, dass das Testelement vor der Benutzung des analytischen Hilfsmittels mindestens eine Schlaufe aufweist, die lösbar mit dem Grundkörper oder der Abdeckung verbunden ist. Vorzugsweise umfasst das an dem Grundkörper befestigte Testelement vor Gebrauch zwei Schlaufen, die auf beiden Seiten neben dem Testfeld nur leicht angeheftet sind. Dies hat den Vorteil, dass das Testelement vor Gebrauch des erfindungsgemäßen analytischen Hilfsmittels platzsparend zusammengelegt ist und nicht flattert. Zum Gebrauch kann es dann von dem Grundkörper an den lösbaren Stellen abgelöst und in seiner vollen Länge entfaltet werden.

Die Erfindung bezieht sich weiterhin auf ein Analysegerät zur Analyse einer Probe mittels eines erfindungsgemäßen analytischen Hilfsmittels. Das Analysegerät umfasst zwei Halteelemente zum Aufnehmen der zwei Teilkörper des zerbrochenen Grundkörpers des analytischen Hilfsmittels und einen Messkopf, über den das zumindest teilweise flexible Testelement spannbar ist. Der Messkopf besitzt vorzugsweise die Form eines Fingers und enthält (z.B. in der Spitze des Fingers) eine Messanordnung zur Analyse einer Probe, die sich auf dem Testfeld eines an dem Messkopf anliegenden Testelements befindet. Zur Analyse der Probe können z.B. optische Verfahren (wie die Reflexionsphotometrie, Absorptionsmessung oder Fluoreszenzmessung) oder elektrochemische Verfahren (wie die Potentiometrie, die Amperometrie, die Voltametrie oder die Coulometrie) zur Anwendung kommen.

Vorzugsweise sind die Halteelemente und der Messkopf parallel zueinander verschiebbar. Dadurch kann z.B. das Halteelement, das den Teilkörper fixiert, der mit der Lanzette verbunden ist, so verschoben werden, dass die Lanzettenspitze eine Stechbewegung ausführt. Dabei kann der andere Teilkörper mit dem anderen Halteelement in der entgegengesetzten Richtung verschoben werden, um eine gewisse Spannung des über den Messkopf gespannten Testelementes aufrecht zu erhalten. Nach dem Einstich der Lanzettenspitze in die Haut eines Anwenders zur Erzeugung einer Perforation kann der (z.B. fingerförmige) Messkopf in Richtung der Perforation verschoben werden, um eine Probe (Blut oder interstitielle Flüssigkeit) aufnehmen zu können.

Gemäß einer bevorzugten Ausführungsform der vorliegenden Erfindung umfasst das erfindungsgemäße Analysegerät eine Biegeeinrichtung zum Zerbrechen des Grundkörpers des analytischen Hilfsmittels vor Gebrauch in die zwei Teilkörper durch Aufbringen einer Biegekraft auf den Grundkörper. Beispielsweise kann die Biegeeinrichtung ein Verschluss des Analysegerätes sein, der nach dem Einsetzen eines erfindungsgemäßen analytischen Hilfsmittels in eines der Halteelemente zugeklappt wird und dabei so mit dem Messkopf zusammenwirkt, dass der Grundkörper über den Messkopf gebogen wird, bis er an der Sollbruchstelle in die zwei Teilkörper zerbricht.

Ein Verfahren zur Analyse einer Probe mit Hilfe eines erfindungsgemäßen analytischen Hilfsmittels erfolgt mit den Schritten
A) Einsetzen eines Endes des analytischen Hilfsmittels in ein erstes Halteelement in einem Analysegerät,
B) Zerbrechen des Grundkörpers des analytischen Hilfsmittels in einen ersten und einen zweiten Teilkörper und Öffnen der Abdeckung, wobei die Lanzettenspitze zur Benutzung freigegeben wird, indem sie mit dem ersten Teilkörper fest verbunden bleibt und über diesen hinausragt,
C) Spannen des zumindest teilweise flexibel ausgebildeten Testelements, das an beiden Teilkörpern befestigt ist, über einen in dem Analysegerät enthaltenen Messkopf, wobei der erste Teilkörper in dem ersten Halteelement und der zweite Teilkörper in einem zweiten Halteelement in dem Analysegerät fixiert ist,
D) Entnahme der Probe durch Einstich der Lanzettenspitze an einer Proben-Entnahmestelle und Aufnahme der Probe auf das über den Messkopf gespannte Testelement, so dass die Probe auf ein Testfeld des Testelements gelangt und
E) Analyse der in dem Testfeld vorhandenen Probe mittels des Messkopfes.

Das Einsetzen des analytischen Hilfsmittels in Schritt A) des Verfahrens kann automatisch oder manuell erfolgen. Das Einsetzen kann z.B. durch Stecken eines Endes des Grundkörpers in ein eine entsprechende Vertiefung aufweisendes erstes Halteelement erfolgen.

Das Zerbrechen des analytischen Hilfsmittels gemäß Schritt B) erfolgt vorzugsweise durch Aufbringen einer Biegekraft auf den Grundkörper des analytischen Hilfsmittels, so dass er an der Sollbruchstelle in die zwei Teilkörper zerbricht. Die Abdeckung wird dabei ebenfalls zerteilt, entweder auch durch Zerbrechen aufgrund der Biegekraft oder sie wird durch die Lanzette aufgeschnitten, die über die Bruchkante des ersten Teilkörpers hinausragt und nicht zerbricht. Die Biegekraft kann zum Beispiel durch eine Biegeeinrichtung des Analysegeräts oder manuell aufgebracht werden.

Durch das Spannen des Testelements in Schritt C) werden gegebenenfalls vorhandene Schlaufen des Testelements gelöst und das auf dem Testelement vorhandene Testfeld in eine oder in die Nähe einer Messposition gebracht. Vorzugsweise wird also beim Spannen des Testelementes in Schritt C) die mindestens eine Schlaufe des Testelements von den Teilkörper gelöst.

Der Einstich der Lanzettenspitze in eine Proben-Entnahmestelle (z.B. die Haut der Fingerbeere) in Schritt D) erfolgt z.B. durch eine automatische Vor- und Zurückbewegung der Lanzettenspitze durch eine Öffnung in dem Gehäuse des Analysegeräts. Anschließend wird der Messkopf mit dem darüber gespannten Testelement ebenfalls durch die Öffnung in dem Gehäuse des Analysegeräts bewegt und in eine exponierte Lage gebracht, in der die Probe auf einen Aufnahmeort auf dem Testelement gelangt, der entweder direkt auf oder an dem Testfeld liegt, oder so gestaltet ist, dass ein Transport der Probe zu dem Testfeld sichergestellt ist (beispielsweise durch eine Kapillare). Es werden also vorzugsweise der Messkopf mit dem darüber gespannten Testelement und die Teilkörper gegeneinander verschoben, um in Schritt D) zunächst die Lanzette zur Proben-Entnahmestelle zu bewegen und dann den Messkopf in Position zur Aufnahme der Probe zu bringen.

Die Analyse der Probe in Schritt E) wird vorzugsweise mittels eines der oben genannten optischen oder elektrochemischen Analyseverfahren durchgeführt, wobei sich das Testfeld des Testelements in einer Messposition befindet.

### Zeichnung

Anhand der Zeichnung wird die Erfindung nachstehend näher erläutert. Es zeigt
- Fig. 1A: eine Schnittdarstellung eines Grundkörpers und einer daran befestigten Lanzette eines erfindungsgemäßen analytischen Hilfsmittels von der Seite,
- Fig. 1 B: eine Schnittdarstellung eines Grundkörpers und einer daran befestigten Lanzette eines erfindungsgemäßen analytischen Hilfsmittels von oben,
- Fig. 1C: eine Lanzette für ein erfindungsgemäßes analytisches Hilfsmittel,
- Fig. 2: eine Schnittdarstellung eines erfindungsgemäßen analytischen Hilfsmittels von der Seite,
- Fig. 3A: eine schematische Darstellung eines Ausschnitts aus einem erfindungsgemäßen Analysegeräts mit einem eingesetzten erfindungsgemäßen analytischen Hilfsmittel und
- Fig. 3B: den Ausschnitt aus Fig. 3A, wobei der Grundkörper des analytischen Hilfsmittels in zwei Teilkörper zerbrochen und das Testelement über einen Messkopf gespannt ist.

### Ausführungsvarianten

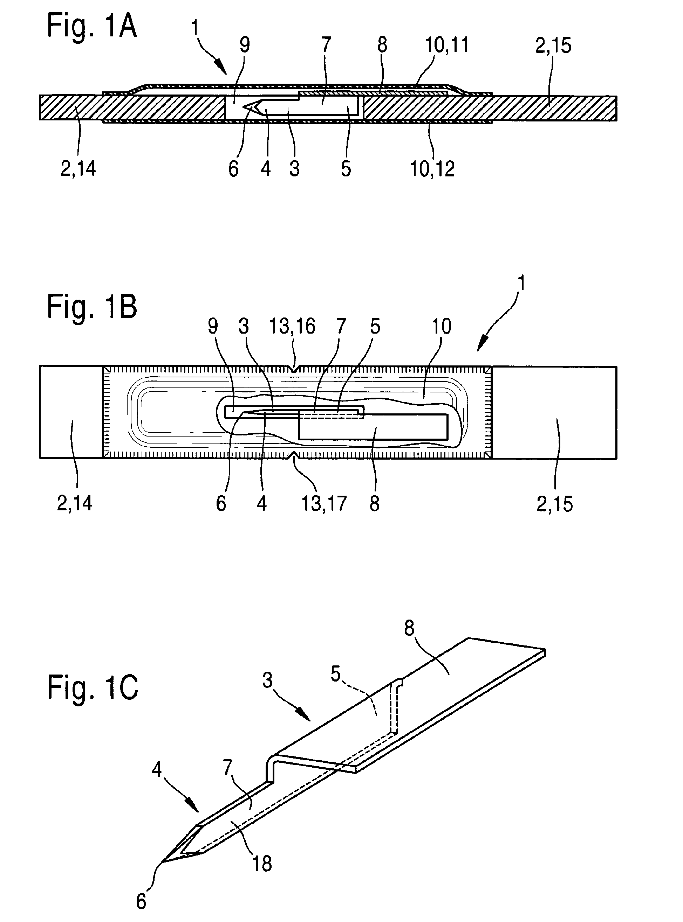

Figuren 1A und 1B zeigen eine Schnittdarstellung eines Grundkörpers und einer daran befestigten Lanzette eines erfindungsgemäßen analytischen Hilfsmittels von der Seite bzw. von oben.

Das analytische Hilfsmittel 1 enthält einen Grundkörper 2 und eine Lanzette 3. Der Grundkörper 2 ist z.B. ein Kunststoffträger. Zusätzlich enthält das analytische Hilfsmittel gemäß der vorliegenden Erfindung ein Testelement, das jedoch nicht in Fig. 1A und 1B dargestellt ist.

Die Lanzette 3 weist ein proximales Ende 4 und ein distales Ende 5 auf. Das proximale Ende 4 ist als Lanzettenspitze 6 ausgebildet. Das distale Ende 5 ist fest mit dem Grundkörper 2 verbunden. Lanzette 3 enthält einen das proximale Ende 4 umfassenden ersten Teilabschnitt 7 und einen mit dem distalen Ende 5 verbundenen zweiten Teilabschnitt 8, wobei der erste Teilabschnitt 7 in einem Winkel von 90 °zu dem zweiten Teilabschnitt 8 angeordnet ist. Dies ist insbesondere in Fig. 1C zu erkennen. Der zweite Teilabschnitt 8 ist fest mit dem Grundkörper 2 verbunden. Diese Verbindung kann bei der Herstellung des analytischen Hilfsmittels 1 z.B. durch Verkleben, Verschweißen, Verklipsen oder Verschrauben erzeugt werden. Der erste Teilabschnitt 7 (inklusive Lanzettenspitze 6) der Lanzette 3 ist bei dem analytischen Hilfsmittel 1 gemäß Fig. 1A in einer Ausnehmung 9 in dem Grundkörper eingebettet. Die Ausnehmung 9 ist ein mittig und in Längsrichtung in dem Grundkörper 2 angeordneter, von oben nach unten durchgehender Schlitz, der zur Aufnahme des scharf geschliffenen Teils der Lanzette 3 dient, solange das analytische Hilfsmittel 1 gelagert und noch nicht verwendet wird. Die Ausnehmung 9 ist beidseitig mit einer Abdeckung 10 verschlossen. Diese Abdeckung 10 besteht aus zwei dünnen Folien 11, 12, mit denen die Ober- und Unterseite des Grundkörpers 2 versiegelt werden. Durch diese Abdeckung 10 wird gewährleistet, dass die zuvor sterilisierte Lanzettenspitze 6 in der verschlossenen Ausnehmung 9 bis kurz vor der Verwendung des analytischen Hilfsmittels 1 steril verwahrt bleibt.

Der Grundkörper 2 weist ferner eine Sollbruchstelle 13 auf, an der der Grundkörper 2 bei Aufbringen einer Spannung (insbesondere einer Biegespannung) in zwei Teilkörper 14, 15 zerbricht. Die Sollbruchstelle 13 wird durch seitliche Einkerbungen 16, 17 im Grundkörper 2 vorgegeben. Bei Aufbringen einer Biegespannung auf den Grundkörper 2 bricht dieser entlang einer Bruchkante, die von der einen Einkerbung 16 zu der anderen Einkerbung 17 verläuft, in zwei Teilkörper 14, 15, ohne die Lanzette 3 zu beschädigen. Die Sollbruchstelle 13 ist dabei so angeordnet, dass die Lanzettenspitze 6 beim Zerbrechen des Grundkörpers 2 in die zwei Teilkörper 14, 15 zur Benutzung freigegeben wird. Dies erfolgt durch ein Zerschneiden oder Zerbrechen der Abdeckung 10, so dass die Lanzettenspitze 6 über die Bruchkante des zweiten Teilkörpers 15 hinausragt.

Die Abdeckung 10 ist im Bereich der Sollbruchstelle 13 innig mit dem Grundkörper 2 verbunden. Sowohl die Abdeckung 10 als auch der Grundkörper 2 bestehen vorzugsweise aus einem Material, das durch die Einwirkung von γ-Strahlung stark versprödet. Dies erlaubt eine Fertigung von der Rolle und ermöglicht trotzdem beim Gebrauch des analytischen Hilfsmittels 1 ein einfaches Auseinanderbrechen des Grundkörpers 2 mitsamt den als Abdeckung 10 dienenden Folien.

Fig. 1C zeigt eine Lanzette für ein erfindungsgemäßes analytisches Hilfsmittel, wie sie in dem analytischen Hilfsmittel gemäß Figuren 1A und 1B verwendet wird.

Die Lanzette 3 umfasst zwei Teilabschnitte 7, 8, die in einem Winkel von 90 ° zueinander angeordnet sind. Der erste Teilabschnitt 7 umfasst eine messerartige Klinge 18 (Flachlanzette), die sich zur Lanzettenspitze 6 hin verjüngt. Beim Zerbrechen des den ersten Teilabschnitt 7 der Lanzette 3 im analytischen Hilfsmittel 1 umgebenden Grundkörpers 2 kann die Klinge 18 die als Abdeckung 10 dienenden Folien durchschneiden. Der zweite Teilabschnitt 8 der Lanzette dient zu ihrer Befestigung am Grundkörper 2 eines erfindungsgemäßen analytischen Hilfsmittels 1.

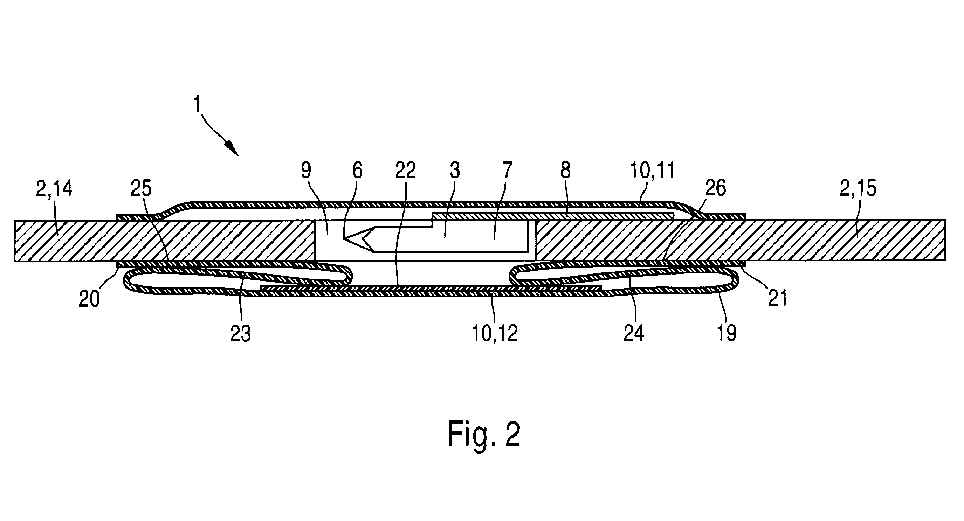

Fig. 2 zeigt eine Schnittdarstellung eines erfindungsgemäßen analytischen Hilfsmittels von der Seite.

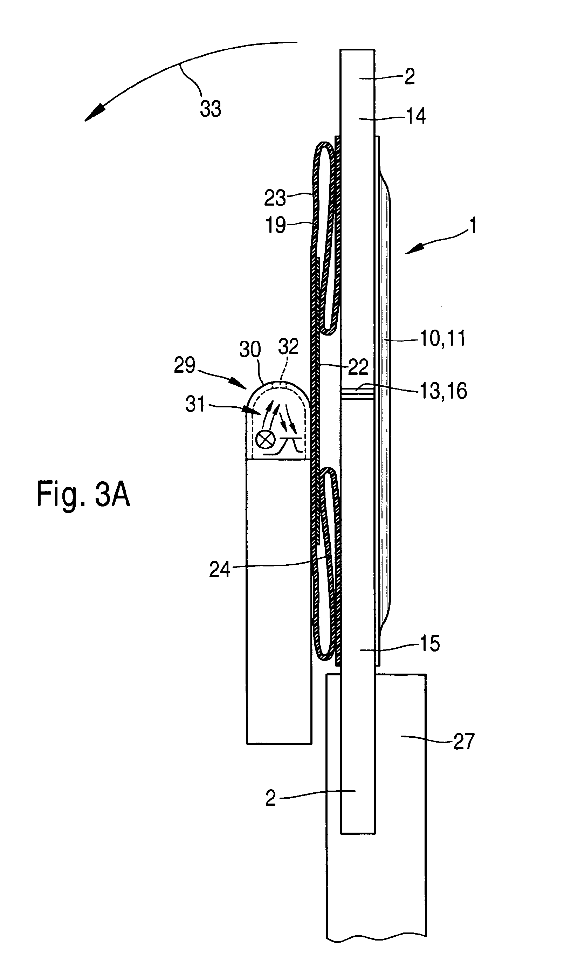

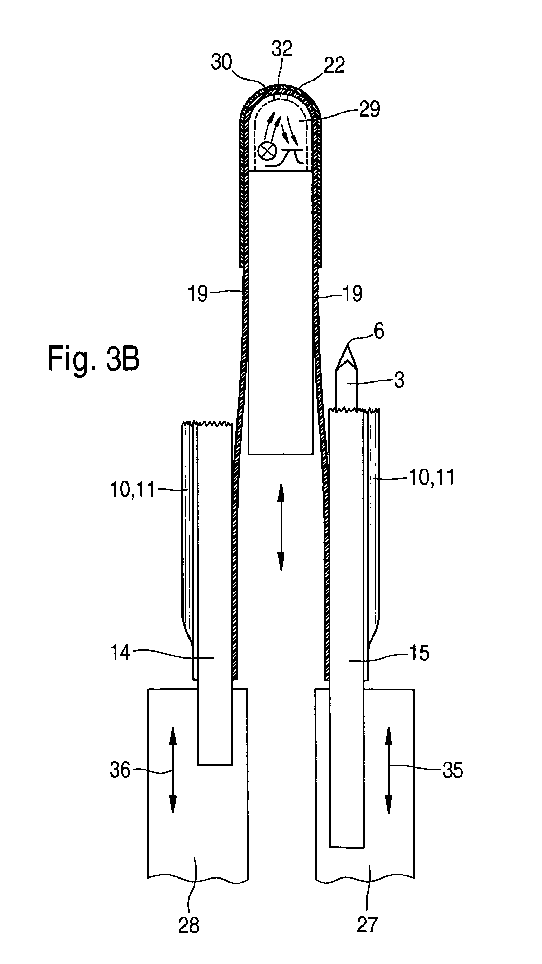

Der Aufbau des in Fig. 2 dargestellten analytischen Hilfsmittels 1 entspricht dem bezüglich Fig. 1A bis 1C bereits beschriebenen Aufbau. Zusätzlich ist in Fig. 3 ein Testelement 19 dargestellt. Das Testelement 19 ist an zwei Enden 20, 21 fest mit dem Grundkörper 2 verbunden. Das Testelement 19 besteht aus einem flexiblen Material und es enthält ein Testfeld 22 mit einer Testchemie. Das Testfeld 22 ist dem Grundkörper 2 zugewandt. Bei diesem analytischen Hilfsmittel 1 ist es vorgesehen, die Probe (z.B. Blut) direkt auf das Testfeld 22 aufzutragen. Das Testfeld 22 entspricht also der Auftragfläche zum Auftragen der zu analysierenden Probe.

Das Testelement wird aus einem flexiblen Testband hergestellt. Es ist ein Abschnitt eines solchen flexiblen Testbandes, der ein Testfeld 22 enthält. Das Testelement 19 weist zwei Schlaufen 23, 24 auf, die lösbar an den Verbindungsstellen 25, 26 mit dem Grundkörper verbunden sind. Bei der Herstellung des Testelements 19 aus einem Textbandabschnitt wird dieser so gefaltet und an dem Grundkörper 2 (bzw. der Abdeckung 10) befestigt, dass die Enden des Bandabschnittes fest mit dem analytischen Hilfsmittel 1 verbunden sind (z.B. durch Verkleben), während die beiden in Schlaufen 23, 24 gelegten langen Stücke des Bandabschnittes links und rechts vom Testfeld 22 nur leicht angeheftet sind, so dass sie am Flattern gehindert werden und leicht abgelöst werden können.

Die in Fig. 1A-1C gezeigte Komponente des analytischen Hilfsmittels gemäß Fig. 2 (Grundkörper 2, Lanzette 3, Abdeckung 10) kann separat von dem Testelement 19 gefertigt werden, so dass die Testchemie des Testfeldes 22 nicht durch die Sterilisierung der Lanzette 3 negativ beeinflusst wird.

Fig. 3A zeigt eine schematische Darstellung eines Ausschnitts aus einem erfindungsgemäßen Analysegerät mit einem eingesetzten erfindungsgemäßen analytischen Hilfsmittel.

Der gezeigte Ausschnitt befindet sich innerhalb eines (nicht dargestellten) Gehäuses des Analysegeräts und ist daher für einen Anwender von außen nicht sichtbar. Das Analysegerät dient zur Analyse einer Probe mittels eines erfindungsgemäßen analytischen Hilfsmittels durch ein optisches Analyseverfahren. Das Analysegerät umfasst zwei Halteelemente 27, 28 zum Aufnehmen der zwei Teilkörper 14, 15 des Grundkörpers 2 eines erfindungsgemäßen analytischen Hilfsmittels 1. Des Weiteren enthält das Analysegerät einen Messkopf 29, über den das verbliebene Testelement 19 spannbar ist und der während der Perforation der Haut eines Anwenders mit der Lanzette 3 und während der Probenaufnahme zwischen den zwei Halteelementen 27, 28 angeordnet ist.

Fig. 3A zeigt das erfindungsgemäße analytische Hilfsmittel 1, das von einem Anwender mit einem Ende in das erste Halteelement 27 des Analysegeräts eingesetzt wurde. Das erste Halteelement 27 ist eine Art Klammer, die das Ende des analytischen Hilfsmittels 1 umschließt und es so fixiert. Der fingerförmige Messkopf 29 ist dabei parallel zu dem analytischen Hilfsmittel angeordnet. Sein abgerundetes Ende 30 befindet sich in dieser Position auf der Höhe der Sollbruchstelle 13 des Grundkörpers 2. In dem abgerundeten Ende 30 des Messkopfes 29 befindet sich eine Messoptik 31 (z.B. in Form einer Licht emittierenden Diode und einer Photodiode), die über ein transparentes Fenster 32 in der Spitze des Messkopfes 29 mit der Außenseite (insbesondere mit einem Analyten auf einem Testfeld) wechselwirken kann.

Der Grundkörper des erfindungsgemäßen analytischen Hilfsmittels 1 wird über das abgerundete Ende 30 des Messkopfes 29 in Pfeilrichtung 33 gebogen, bis er an der Sollbruchstelle 13 in die zwei Teilkörper 14, 15 zerbricht. Dabei wird die Abdeckung 10 (Folien) geöffnet, entweder durch Zerbrechen aufgrund der Biegespannung oder durch Zerschneiden durch die Lanzette 3. Dadurch wird die Lanzettenspitze 6 zur Benutzung freigegeben. Sie ragt über die Bruchkante des ersten Teilkörpers 14 hinaus und bleibt mit diesem verbunden (siehe Fig. 3B). Das Durchbiegen zum Zerbrechen des Grundkörpers 2 erfolgt mittels einer in dem Analysegerät enthaltenen (nicht dargestellten) Biegeeinrichtung. Dabei kann es sich z.B. um eine Klappe des Analysegeräts handeln, die der Anwender nach dem Einsetzen des analytischen Hilfsmittels 1 in das Analysegerät manuell schließt und die beim Schließen eine Kraft in Pfeilrichtung 33 auf den Grundkörper 2 ausübt. Gleichzeitig gleitet das von dem ersten Halteelement 27 abgewandte Ende des Grundkörpers 2 in ein (in Fig. 3A nicht dargestelltes) zweites Halteelement 28, das wie das erste Halteelement 27 ausgebildet ist und nach dem Zerbrechen des Grundkörpers 2 den ersten Teilkörper 14 fixiert.

Fig. 3B zeigt den Ausschnitt des Analysegeräts aus Fig. 3A, wobei der Grundkörper des analytischen Hilfsmittels in zwei Teilkörper zerbrochen und das Testelement über den Messkopf gespannt ist.

Die beiden Teilkörper 14, 15 werden jeweils durch ein Halteelement 27, 28 fixiert. Das in Fig. 3A zusammengefaltet dargestellte Testelement 19 ist über den Messkopf 29 gespannt. Der Messkopf 29 ist in Richtung 34 federnd ausgebildet, damit stets eine Grundspannung des über den Messkopf 29 gebogenen Testelements 19 aufrecht erhalten wird. Beim Spannen des Testelements 19 über den Messkopf 29 werden die zwei Schlaufen 23, 24 von den Teilkörpern 14, 15 gelöst und das Testelement 19 in seiner vollen Länge entfaltet. Die Halteelemente 27, 28 und der Messkopf 29 sind parallel zueinander verschiebbar. Dadurch kann zunächst die Lanzette 3 zur Proben-Entnahmestelle bewegt und dann der Messkopf 29 in Position zur Aufnahme der Probe gebracht werden. Die Halteelemente 27, 28 werden gegenphasig zueinander mittels eines (nicht dargestellten) Antriebs in den Richtungen 35, 36 verschoben (wie eine Wippe), so dass das Testelement 19 dabei gespannt bleibt.

Zur Entnahme einer Probe wird das erste Halteelement 27 (in Fig. 3B nach oben) verschoben, so dass die Lanzettenspitze kurzzeitig durch eine (nicht dargestellte) Öffnung im Gehäuse des Analysegerätes austritt und dort die Haut eines Anwenders an einer Proben-Entnahmestelle perforiert, z.B. die Haut einer dort angelegten Fingerbeere. Dann wird das erste Halteelement 27 mit dem daran fixierten Teilkörper 15 und der Lanzette 3 wieder vollständig in das Gehäuse des Analysegeräts zurückgezogen. Das zweite Halteelement 28 mit dem ersten Teilkörper 14 führt eine der Bewegung des ersten Halteelements 27 entgegengesetzte Bewegung aus, damit das Testelement 19 über den Messkopf 29 gespannt bleibt. Anschließend führt der Messkopf 29 (und damit auch die Halteelemente 27, 28 mit den Bestandteilen des analytischen Hilfsmittels 1) eine seitliche Bewegung oder Schwenkbewegung aus, so dass der Messkopf 29 durch die Öffnung im Gehäuse des Analysegeräts zur Entnahmestelle geschoben werden kann. Dies kann beispielsweise durch ein Verschieben des Messkopfes 29 mitsamt den Halteelementen 27, 28 und den Bestandteilen des analytischen Hilfsmittels 1 oder des Messkopfes 29 alleine zu der Öffnung hin (in Fig. 3B nach oben) erfolgen. Sobald der Messkopf 29 durch die Öffnung aus dem Gehäuse des Analysegeräts herausragt, wird die Probe (Blut oder interstitielle Flüssigkeit) auf das über den Messkopf 29 gespannte Testelement 19 übertragen, so dass sie auf ein Testfeld 22 des Testelements 19 gelangt. Dabei wird die Probe vorzugsweise direkt auf das auf dem abgerundeten Ende 30 des Messkopfes 29 angeordnete Testfeld 22 gegeben. In diesem Fall ist keine Kapillarspalte zum Transport der Probe zum Testfeld erforderlich, so dass keine Probleme mit der Hydrophilie bestehen. Ferner handelt es sich in diesem Fall um eine Flächendosierung, was die Aufnahme der Probe toleranzunempfindlicher macht. Es besteht ferner die Möglichkeit, mit dem Testfeld 22 mit einer geringen Kraft gegen die Hautoberfläche an der Proben-Entnahmestelle zu fahren, um die Probenübernahme auf das Testfeld 22 zu sichern. Ein erneuter Einstich der Lanzettenspitze 6 in die Haut kann mit dem erfindungsgemäßen Analysegerät in vorteilhafter Weise vermieden werden.

Die auf dem Testfeld 22 vorhandene Probe wird anschließend mittels der Messoptik 31 in dem Messkopf 29 analysiert.

### Bezugszeichenliste

- 1: analytisches Hilfsmittel
- 2: Grundkörper
- 3: Lanzette
- 4: proximales Ende der Lanzette
- 5: distales Ende der Lanzette
- 6: Lanzettenspitze
- 7: erster Teilabschnitt der Lanzette
- 8: zweiter Teilabschnitt der Lanzette
- 9: Ausnehmung
- 10: Abdeckung
- 11: erste Folie
- 12: zweite Folie
- 13: Sollbruchstelle
- 14: erster Teilkörper
- 15: zweiter Teilkörper
- 16: erste seitliche Einkerbung
- 17: zweite seitliche Einkerbung
- 18: messerartige Klinge
- 19: Testelement
- 20: erstes Ende des Testelementes
- 21: zweites Ende des Testelements
- 22: Testfeld
- 23: erste Schlaufe des Testelementes
- 24: zweite Schlaufe des Testelementes
- 25: erste Verbindungsstelle
- 26: zweite Verbindungsstelle
- 27: erstes Halteelement
- 28: zweites Halteelement
- 29: Messkopf
- 30: abgerundetes Ende des Messkopfes
- 31: Messoptik
- 32: Fenster
- 33: Pfeilrichtung
- 34: Verschiebungsrichtung des Messkopfes
- 35: Verschiebungsrichtung des ersten Halteelementes
- 36: Verschiebungsrichtung des zweiten Halteelementes

## Patentansprüche

1. Analytisches Hilfsmittel enthaltend einen Grundkörper (2), eine Lanzette (3) und ein Testelement (19),
- wobei die Lanzette (3) ein proximales (4) und ein distales (5) Ende aufweist, wobei das proximale Ende (4) der Lanzette (3) als Lanzettenspitze (6) ausgebildet ist und das distale Ende (5) der Lanzette (3) fest mit dem Grundkörper (2) verbunden ist und wobei zumindest die Lanzettenspitze (6) in einer Ausnehmung (9) in dem Grundkörper (2) eingebettet und die Ausnehmung (9) mit einer Abdeckung (10) verschlossen ist, solange das analytische Hilfsmittel (1) sich in einem unbenutzten Zustand befindet ,
- wobei das Testelement (19) fest mit dem Grundkörper (2) verbunden ist,
**dadurch gekennzeichnet, dass**
- der Grundkörper (2) eine Sollbruchstelle (13) aufweist, an der der Grundkörper (2) bei Aufbringen einer Spannung in zwei Teilkörper (14, 15) zerbricht, wobei die Sollbruchstelle (13) so angeordnet ist, dass die Lanzettenspitze (6) beim Zerbrechen des Grundkörpers (2) an der Sollbruchstelle (13) zur Benutzung freigegeben wird,

2. Analytisches Hilfsmittel gemäß Anspruch 1, **dadurch gekennzeichnet, dass** die Ausnehmung (9) in dem Grundkörper (2) ein in Längsrichtung verlaufender länglicher Schlitz ist.

3. Analytisches Hilfsmittel gemäß einem der Ansprüche 1 oder 2, **dadurch gekennzeichnet, dass** die Lanzette (3) eine messerartige Klinge umfasst, die sich zur Lanzettenspitze (6) hin verjüngt.

4. Analytisches Hilfsmittel gemäß einem der Ansprüche 1 bis 3, **dadurch gekennzeichnet, dass** die Lanzette (3) einen das proximale Ende (4) umfassenden ersten Teilabschnitt (7) und einen mit dem distalen Ende (5) verbundenen zweiten Teilabschnitt (8) enthält, wobei der erste Teilabschnitt (7) abgewinkelt zu dem zweiten Teilabschnitt (8) ausgebildet ist.

5. Analytisches Hilfsmittel gemäß Anspruch 4, **dadurch gekennzeichnet, dass** der zweite Teilabschnitt (8) der Lanzette (3) mit dem Grundkörper (2) fest verbunden ist.

6. Analytisches Hilfsmittel gemäß einem der Ansprüche 4 oder 5, **dadurch gekennzeichnet, dass** der erste Teilabschnitt (7) in einem Winkel von 90° zu dem zweiten Teilabschnitt (8) angeordnet ist.

7. Analytisches Hilfsmittel gemäß einem der Ansprüche 1 bis 6, **dadurch gekennzeichnet, dass** die Abdeckung (10) mindestens eine Folie umfasst, die mindestens eine Oberfläche des Grundkörpers (2) ganz oder teilweise abdeckt.

8. Analytisches Hilfsmittel gemäß einem der Ansprüche 1 bis 7, **dadurch gekennzeichnet, dass** der Grundkörper (2) und/oder die Abdeckung (10) aus einem Material bestehen, das durch Bestrahlung versprödet.

9. Analytisches Hilfsmittel gemäß einem der Ansprüche 1 bis 8, **dadurch gekennzeichnet, dass** die Sollbruchstelle (13) durch seitliche Einkerbungen (16) und/oder mindestens eine Querrille im Grundkörper (2) vorgegeben ist.

10. Analytisches Hilfsmittel gemäß einem der Ansprüche 1 bis 9, **dadurch gekennzeichnet, dass** das Testelement (19) zumindest teilweise aus einem flexiblen Material besteht.

11. Analytisches Hilfsmittel gemäß einem der Ansprüche 1 bis 10, **dadurch gekennzeichnet, dass** das Testelement (19) ein Abschnitt eines flexiblen Testbandes ist, der ein Testfeld (22) enthält.

12. Analytisches Hilfsmittel gemäß einem der Ansprüche 1 bis 11, **dadurch gekennzeichnet, dass** das Testelement (19) eine Auftragfläche zum Auftragen einer zu analysierenden Probe enthält, die vor einer Benutzung des analytischen Hilfsmittels (1) dem Grundkörper (2) zugewandt ist.

13. Analytisches Hilfsmittel gemäß einem der Ansprüche 1 bis 12, **dadurch gekennzeichnet, dass** das Testelement (19) an zwei Enden (20, 21) fest mit dem Grundkörper (2) verbunden ist.

14. Analytisches Hilfsmittel gemäß einem der Ansprüche 1 bis 13, **dadurch gekennzeichnet, dass** das erste Ende (20) des Testelements (19) mit dem ersten Teilkörper (14) und das zweite Ende (21) des Testelements (19) mit dem zweiten Teilkörper (15) fest verbunden ist.

15. Analytisches Hilfsmittel gemäß einem der Ansprüche 1 bis 14, **dadurch gekennzeichnet, dass** das Testelement (19) vor der Benutzung des analytischen Hilfsmittels (1) mindestens eine Schlaufe (23, 24) aufweist, die lösbar mit dem Grundkörper (2) oder mit der Abdeckung (10) verbunden ist.

16. Analysegerät zur Analyse einer Probe mittels eines analytischen Hilfsmittels (1) gemäß einem der Ansprüche 1 bis 15 mit zwei Halteelementen (27, 28) zum Aufnehmen der zwei Teilkörper (14, 15) des Grundkörpers (2) des analytischen Hilfsmittels (1) und mit einem Messkopf (29), über den das zumindest teilweise flexible Testelement (19) spannbar ist.

17. Analysegerät gemäß Anspruch 16, **dadurch gekennzeichnet, dass** die Halteelemente (27, 28) und der Messkopf (29) parallel zueinander verschiebbar sind.

18. Analysegerät gemäß einem der Ansprüche 16 oder 17, umfassend eine Biegeeinrichtung zum Zerbrechen des Grundkörpers (2) des analytischen Hilfsmittels (1) vor Gebrauch in die zwei Teilkörper (27,28) durch Aufbringen einer Biegekraft auf den Grundkörper (2).

## Claims

1. Analytical aid comprising a base body (2), a lancet (3) and a test element (19),
- the lancet (3) having a proximal end (4) and a distal end (5), the proximal end (4) of the lancet (3) being formed as a lancet point (6) and the distal end (5) of the lancet (3) being fixedly connected to the base body (2), and at least the lancet point (6) being embedded in a recess (9) in the base body (2) and the recess (9) being closed by a covering (10) as long as the analytical aid (1) is in an unused state,
- the test element (19) being fixedly connected to the base body (2),
**characterized in that**
- the base body (2) has a predetermined breaking point (13), at which the base body (2) breaks up into two part-bodies (14, 15) when stress is applied, the predetermined breaking point (13) being arranged in such a way that the lancet point (6) is exposed for use when the base body (2) breaks up at the predetermined breaking point (13).

2. Analytical aid according to Claim 1, **characterized in that** the recess (9) in the base body (2) is a longitudinal slit running in the longitudinal direction.

3. Analytical aid according to either of Claims 1 and 2, **characterized in that** the lancet (3) comprises a knife-like blade, which tapers toward the lancet point (6).

4. Analytical aid according to one of Claims 1 to 3, **characterized in that** the lancet (3) comprises a first portion (7), comprising the proximal end (4), and a second portion (8), connected to the distal end (5), the first portion (7) being formed such that it is angled away from the second portion (8).

5. Analytical aid according to Claim 4, **characterized in that** the second portion (8) of the lancet (3) is fixedly connected to the base body (2).

6. Analytical aid according to either of Claims 4 and 5, **characterized in that** the first portion (7) is arranged at an angle of 90° in relation to the second portion (8).

7. Analytical aid according to one of Claims 1 to 6, **characterized in that** the covering (10) comprises at least one film, which entirely or partially covers at least one surface of the base body (2).

8. Analytical aid according to one of Claims 1 to 7, **characterized in that** the base body (2) and/or the covering (10) consist of a material that is made brittle by irradiation.

9. Analytical aid according to one of Claims 1 to 8, **characterized in that** the predetermined breaking point (13) is defined by lateral notches (16) and/or at least one transverse channel in the base body (2).

10. Analytical aid according to one of Claims 1 to 9, **characterized in that** the test element (19) consists at least partially of a flexible material.

11. Analytical aid according to one of Claims 1 to 10, **characterized in that** the test element (19) is a portion of a flexible test strip, which includes a testing area (22).

12. Analytical aid according to one of Claims 1 to 11, **characterized in that** the test element (19) includes an application area for applying a sample to be analyzed, which is facing the base body (2) before the analytical aid (1) is used.

13. Analytical aid according to one of Claims 1 to 12, **characterized in that** the test element (19) is fixedly connected to the base body (2) at two ends (20, 21).

14. Analytical aid according to one of Claims 1 to 13, **characterized in that** the first end (20) of the test element (19) is fixedly connected to the first part-body (14) and the second end (21) of the test element (19) is fixedly connected to the second part-body (15).

15. Analytical aid according to one of Claims 1 to 14, **characterized in that**, before the analytical aid (1) is used, the test element (19) has at least one loop (23, 24), which is detachably connected to the base body (2) or to the covering (10).

16. Analyzer for the analysis of a sample by means of an analytical aid (1) according to one of Claims 1 to 15 comprising two holding elements (27, 28) for receiving the two part-bodies (14, 15) of the base body (2) of the analytical aid (1) and comprising a measuring head (29), over which the at least partially flexible test element (19) can be stretched.

17. Analyzer according to Claim 16, **characterized in that** the holding elements (27, 28) and the measuring head (29) are displaceable parallel to one another.

18. Analyzer according to either of Claims 16 and 17, comprising a bending device for breaking up the base body (2) of the analytical aid (1) before use into the two part-bodies (14, 15) by applying a bending force to the base body (2).

## Revendications

1. Moyen d'analyse comprenant un corps de base (2), une lancette (3) et un élément d'essai (19),
- dans lequel la lancette (3) présente une extrémité proximale (4) et une extrémité distale (5), dans lequel l'extrémité proximale (4) de la lancette (3) est formée en pointe de lancette (6) et l'extrémité distale (5) de la lancette (3) est solidaire du corps de base (2), et dans lequel au moins la pointe de lancette (6) est logée dans une cavité (9) dans le corps de base (2) et la cavité (9) est fermée avec un couvercle (10) aussi longtemps que le moyen d'analyse (1) se trouve dans une position inutilisée,
- dans lequel le moyen d'essai (19) est solidaire du corps de base (2),
**caractérisé en ce que**
- le corps de base (2) présente une zone destinée à la rupture (13) dans laquelle le corps de base (2) se brise en deux corps partiels (14, 15) lors de l'application d'une contrainte, dans lequel la zone destinée à la rupture (13) est disposée de telle manière que la pointe de lancette (6) soit dégagée en vue de l'utilisation lors de la rupture du corps de base (2) dans la zone destinée à la rupture (13).

2. Moyen d'analyse selon la revendication 1, **caractérisé en ce que** la cavité (19) dans le corps de base (2) est une fente allongée s'étendant en direction longitudinale.

3. Moyen d'analyse selon l'une quelconque des revendications 1 ou 2, **caractérisé en ce que** la lancette (3) comprend une lame en forme de couteau, qui se rétrécit en direction de la pointe de lancette (6).

4. Moyen d'analyse selon l'une quelconque des revendications 1 à 3, **caractérisé en ce que** la lancette (3) comporte une première section partielle (7) comprenant l'extrémité proximale (4) et une deuxième section partielle (8) reliée à l'extrémité distale (5), dans lequel la première section partielle (7) est pliée par rapport à la deuxième section partielle (8).

5. Moyen d'analyse selon la revendication 4, **caractérisé en ce que** la deuxième section partielle (8) de la lancette (3) est solidaire du corps de base (2).

6. Moyen d'analyse selon l'une quelconque des revendications 4 ou 5, **caractérisé en ce que** la première section partielle (7) est disposée sous un angle de 90° par rapport à la deuxième section partielle (8).

7. Moyen d'analyse selon l'une quelconque des revendications 1 à 6, **caractérisé en ce que** le couvercle (10) comprend au moins une feuille, qui recouvre entièrement ou partiellement au moins une surface du corps de base (2).

8. Moyen d'analyse selon l'une quelconque des revendications 1 à 7, **caractérisé en ce que** le corps de base (2) et/ou le couvercle (10) sont composés d'un matériau, qui se fragilise par irradiation.

9. Moyen d'analyse selon l'une quelconque des revendications 1 à 8, **caractérisé en ce que** la zone destinée à la rupture (13) est prédéterminée par des encoches latérales (16) et/ou au moins une rayure transversale dans le corps de base (2).

10. Moyen d'analyse selon l'une quelconque des revendications 1 à 9, **caractérisé en ce que** l'élément d'essai (19) se compose au moins partiellement d'un matériau flexible.

11. Moyen d'analyse selon l'une quelconque des revendications 1 à 10, **caractérisé en ce que** l'élément d'essai (19) est une section d'une bande d'essai flexible, qui comprend un champ d'essai (22).

12. Moyen d'analyse selon l'une quelconque des revendications 1 à 11, **caractérisé en ce que** l'élément d'essai (19) comporte une face de dépôt destinée au dépôt d'un échantillon à analyser, qui est tournée vers le corps de base (2) avant une utilisation du moyen d'analyse (1).

13. Moyen d'analyse selon l'une quelconque des revendications 1 à 12, **caractérisé en ce que** l'élément d'essai (19) est solidarisé au corps de base (2) à ses deux extrémités (20, 21).

14. Moyen d'analyse selon l'une quelconque des revendications 1 à 13, **caractérisé en ce que** la première extrémité (20) de l'élément d'essai (19) est solidarisée au premier corps partiel (14) et la deuxième extrémité (21) de l'élément d'essai (19) est solidarisée au deuxième corps partiel (15).

15. Moyen d'analyse selon l'une quelconque des revendications 1 à 14, **caractérisé en ce que** l'élément d'essai (19) présente, avant l'utilisation du moyen d'analyse (1), au moins une boucle (23, 24), qui est reliée de façon séparable au corps de base (2) ou au couvercle (10).

16. Appareil d'analyse pour l'analyse d'un échantillon au moyen d'un moyen d'analyse (1) selon l'une quelconque des revendications 1 à 15 avec deux éléments de maintien (27, 28) destinés à recevoir les deux corps partiels (14, 15) du corps de base (2) du moyen d'analyse (1) et avec une tête de mesure (29) sur laquelle l'élément d'essai (19) au moins partiellement flexible peut être tendu.

17. Appareil d'analyse selon la revendication 16, **caractérisé en ce que** les éléments de maintien (27, 28) et la tête de mesure (29) sont déplaçables parallèlement les uns aux autres.

18. Appareil d'analyse selon l'une quelconque des revendications 16 ou 17, comprenant un dispositif de flexion destiné à rompre le corps de base (2) du moyen d'analyse (1) avant son utilisation en les deux corps partiels (14, 15) par application d'une force de flexion au corps de base (2).
